**Europäisches Patentamt**

**European Patent Office**

**Office européen des brevets**

(19)

(11) Publication number : **0 339 035 B1**

# (12) EUROPEAN PATENT SPECIFICATION

(45) Date of publication of patent specification : **07.10.92 Bulletin 92/41**

(51) Int. Cl.⁵ : **A61K 9/22**

(21) Application number : **88900388.5**

(22) Date of filing : **23.12.87**

(86) International application number : **PCT/GB87/00919**

(87) International publication number : **WO 88/04923 14.07.88 Gazette 88/15**

(54) **HYDROGEL-CONTAINING ENVELOPES.**

(30) Priority : **24.12.86 GB 8630876**

(43) Date of publication of application : **02.11.89 Bulletin 89/44**

(45) Publication of the grant of the patent : **07.10.92 Bulletin 92/41**

(84) Designated Contracting States :
**AT BE CH DE FR GB IT LI LU NL SE**

(56) References cited :
**EP-A- 0 121 331**
**EP-A- 0 132 384**
**US-A- 4 207 890**
**US-A- 4 434 153**

(73) Proprietor : **BRITISH TECHNOLOGY GROUP LTD**
**101 Newington Causeway**
**London SE1 6BU (GB)**

(72) Inventor : **GRAHAM, Neil, Bonnette**
**6 Kilmardinny Grove Bearsden**
**Glasgow G61 3NY (GB)**
Inventor : **RASHID, Abdul**
**25 Camphill Avenue**
**Glasgow G41 3AU (GB)**

(74) Representative : **Gallafent, Richard John et al**
**GALLAFENT & CO. 8 Staple Inn**
**London WCIV 7QH (GB)**

EP 0 339 035 B1

## Description

This invention relates to hydrogel-containing devices for sustained release of active ingredient.

It is known to use hydrogels as carriers, excipients, or delivery agents for active, e.g. pharmaceutical or veterinary, ingredients. There are many treatments where sustained release of active ingredient from, for example, orally administered pharmaceutical or veterinary compositions is desirable. There has been much interest in the use of hydrogels in the preparation of sustained release compositions whereby the active ingredient is released gradually from the composition. Thus US-A-3551556 and US-A-3689634 describe sustained release drug-containing compositions comprising the drug and a hydrogel through which the drug is released. Sustained release preparations comprising an active ingredient and a hydrogel have also been described in GB-A-2047093, GB-A-2047094, GB-A-2090264 and GB-A-2108517.

An important property of active ingredient-containing compositions is that, in use of such compositions, the active ingredient should be released effectively and at the required rate. This is particularly important in pharmaceutical and veterinary compositions where the rate of release of active ingredient is often an important feature in treatment.

It is known to provide sustained release devices in the form of hydrogel-containing envelopes. GB-A-2144051 describes hydrogel-containing, water-permeable or porous envelopes with active ingredient in sustained release form, which are particularly useful in the administration of active ingredient over an extended period of time to animals and humans. One of the problems associated with the sustained release of active ingredient is ensuring that the active ingredient can be conveniently administered while at the same time it is retained within the stomach for a sufficient length of time while the active ingredient is being released. According to GB-A-2144051, the envelope is introduced, by being swallowed by the patient or forced down the throat of an animal with the hydrogel contained therein in non-swollen state. On entering the patient's or animal's stomach, the hydrogel in the envelope swells by absorption of water through the envelope walls and accordingly the envelope is caused to swell up within the stomach. The swollen envelope is retained within the stomach during the sustained release of the active ingredient. Thus there is provided a very simple solution to the problem of retention of sustained release compositions in the stomach. Preferably the envelope described also contains active ingredient in sustained release form and, in use, is swallowed by the patient and the active ingredient is released in the stomach. In addition there is described the use of hydrogel-containing envelopes as appetite suppressors in, for example, the treatment of obesity in which case the presence of active ingredient may be unnecessary. The presence of the swollen envelopes in the stomach may have an appetite-reducing effect.

EP-A-0 121 331 discloses envelopes containing particulate water-insoluble hydrogel. The envelope, which may also contain active ingredients, is limp and floppy when dry, to facilitate insertion through the throat, but will swell up in the stomach for sustained release of the active ingredient.

Because of the preferred mode of administration or introduction, there are often practical restrictions on the amount of material which can be included in the envelope. The most convenient method of administration of the envelopes of GB-A-2144051 is of course for them either to be swallowed by the patient or forced down the throat in the case of an animal. Thus the envelope can only contain an amount small enough to allow this. This can be restricting on the amount which can be incorporated within one envelope and accordingly, in the case of sustained release of active ingredient, may be restricting upon the total dose of active ingredient or the length of treatment time for one envelope. One difficulty which can occur is that there may need to be used large amounts of active ingredient (e.g. six months' supply or more) together with carrier or other means for controlling release. If there are practical restrictions on the size of the envelope composition, this can be a limiting factor on the amount of active ingredient which can be administered to the patient at any one time, thus increasing the frequency at which the composition has to be administered.

For example, to provide for release for 200 days at 100mg/day requires 20g active ingredient. This in turn may require about 50g of hydrogel to control the release of active ingredient. At an average powder density of $0.25g.cm^{-3}$ it is not possible to fit the charge into a hard gelatin capsule of the size for example 100mm x 38mm, the maximum for an oral dosage in cattle.

Similarly, where it is desired to have a large swollen envelope or a particularly rigidly inflated swollen envelope e.g. for better retention in the stomach, more hydrogel in the envelope will be required.

According to the present invention there is provided a device for sustained release for active ingredient which device comprises an envelope having flexible water-permeable walls and containing swellable material and active ingredient, characterised in that the envelope has flexible water-permeable and porous walls of perforated plastics, knitted, braided or woven material and contains one or more water-disintegratable, self-coherent compresses of hydrogel particles and contains a sustainedly releasable biologically, veterinarily or pharmaceutically active ingredient.

By use of hydrogel in compress form it is possible to increase the weight of hydrogel which can be included in a particular envelope. When the compressed hydrogel used according to the present invention is placed in an aqueous environment, which can for example simply be water (alone) or can be in the human or an animal stomach, the hydrogel particles first swell and return to their non-compressed form. This initial increase in size of the hydrogel particles results in a rapid disintegration of the formed composition into particle form, a rapid increase in the surface area of the initially small form and a rapid initial swelling of the envelope in which the hydrogel is contained. Moreover, there is a subsequent further increase in volume as the hydrogel particles swell by absorption of water from the environment causing further swelling of the envelope.

Thus, with the hydrogel-compress-containing envelopes according to the present invention, it is possible to provide a device which, for administration or introduction, can be provided in small size but which on rapid swelling provides an envelope of large size and/or with high swelling.

The compresses according to the present invention contain a biologically-, veterinarily- or pharmaceutically-active ingredient. The active ingredient can be in physical mixture with the hydrogel particles or can be chemically bonded to or charged into the matrix of the hydrogel particles used. Alternatively the active ingredient may be separate from the hydrogel compress(es) rather than included in them.

The characteristics of the hydrogel used according to the present invention are particularly advantageous when an active ingredient which is water-insoluble is included in the hydrogel compress(es). The large surface area provided after disintegration of the compressed composition and further swelling of the hydrogel particles provides a composition of large surface area from which active ingredient can be released at a uniform rate.

Because in the envelopes used according to the invention the hydrogel is compressed, the present invention may provide sustained release devices having high content of active ingredient and of restricted size. This is particularly of interest with sustained release devices where high envelope loadings are advantageous and large size may be disadvantageous. It enables there to be provided sustained release devices which comprise a dosage for a considerable time in swallowable form for example.

According to a preferred aspect of the present invention, there is provided a device in which the envelope has a given maximum non-stretched internal volume and the volume of the quantity of hydrogel present, when fully swollen at 20°C, amounts to at least 66%, preferably at least 100%, of the given maximum non-stretched internal volume but insufficient when fully swollen at 20°C to rupture the envelope. Envelopes of this type are as described in GB-A-2144051.

The "maximum non-stretched internal volume" of the envelope used according to the present invention is the maximum internal volume of the envelope before the walls of the envelope start stretching. When a flexible-walled envelope is flat it has an internal volume of substantially zero. As material is introduced into the envelope, e.g. by inflation, the internal volume of the envelope will increase, without stretching of the envelope walls, to a maximum. Beyond this maximum non-stretched internal volume any further increase in the internal volume of the envelope involves stretching of the material of the envelope walls. Eventually, of course, if too much internal pressure is applied the envelope walls will rupture.

Hydrogel particles may be compressed into coherent shaped forms by simply subjecting the particles to pressure in a press.

The compresses according to the invention may be prepared by mixing the ingredients, placing them in a mould and subjecting them to a compacting pressure. The compacting pressure will depend upon the amount of material being compressed and on the degree of compression sought. It may be necessary for special measures to be taken to ensure ready release of the compress from the mould e.g. by use of a mould release agent or by freezing.

Generally the compression ratio will be in the range 3:1 to 5:1.

Preferably the hydrogel used should be one which is fairly highly, swelling; that is the hydrogel (uncompressed) should have a swelling at 25°C of 100 to 800 parts per hundred parts.

The hydrogel used can be a natural or synthetic, organic or inorganic material. Preferably the hydrogel used will be synthetic. Suitable materials are made from water-soluble backbone materials which are rendered insoluble by the introduction of covalent crosslinks e.g. addition polymers of hydroxy alkyl(meth)acrylates, methyl vinyl ether, (meth)acrylamide, N-vinyl pyrrolidone, (meth)acrylic acid and its salts, N-vinyl and C-vinyl pyridines and salts thereof with poly(meth)acrylates such as glycol dimethacrylate. There may also be used crosslinked natural polymers such as collagen or starch and cellulose derivatives and crosslinked synthetic polymers such as polyvinyl alcohol may be used.

Preferably there is used, as hydrogel, a crosslinked poly(ethylene glycol or ethylene oxide). Such materials have been found to compress readily and to provide particularly effective disintegration on contact with water.

Suitably crosslinked materials can be prepared by reacting poly(ethylene glycol) with a polyol (e.g. 1,2,6-hexantriol) and a polyisocyanate (e.g. diphenylmethane 4,4'-diisocyanate or bis-cyclohexylmethylene-4,4'-diisocyanate). Further there may be used materials rendered insoluble by entanglement crosslinking (high molecular weight poly(ethylene oxides)) with divinylbenzene or by crystallinity (cellulosic materials). Also there may suitably be used poly(ethylene glycol) hydrogels based on aromatic and aliphatic isocyanates as well as the biodegradable systems based on monomers containing a multiplicity of 3,4-dihydro-2H-pyran units.

In the compresses used according to the present invention, the active ingredient and hydrogel particles may be in physical mixture. Alternatively the hydrogel particles may contain active ingredient in a sustained release form for example as described in US-A-4150108 and US-A-4221779. Many formulations for the sustained release of active ingredient in this way are known. Thus the active ingredient may be present, in sustained release form, in the hydrogel particles themselves. Suitable such compositions are described in for example GB-A-2047093, GB-A-2047094 and GB-A-2108517.

The manner in which the active ingredient is included in the envelope according to the present invention will depend upon its solubility, physical form, dose rate, size etc. Thus if the active ingredient is itself water-insoluble it may be sufficient to admix the hydrogel and active ingredient to achieve the required sustained release rates. If the active ingredient is water-soluble, then it needs to be used in a form to retard dissolution to the required sustained release rates e.g. by incorporation into the hydrogel particles. Also, as mentioned above, the active ingredient may be separate from the hydrogel compresses. Thus, in the sustained release device according to the invention, the release of the active ingredient may be controlled by means other than the hydrogel. For example the active ingredient may be present within the device according to the invention in its own controlled release device, e.g. a device as described in GB-A-2143733 or as described in GB-A-2153675 or an Alzet device as sold by Alza Corporation of Palo Alto, California, United States of America.

Incorporation of active ingredient into the hydrogel particles may reduce their swellability. Thus it may be desirable to use a mixture of non-active ingredient-containing hydrogel with active-ingredient loaded hydrogel in the compresses according to the invention.

The hydrogel used is in particle form. Suitably the hydrogel particles are of the order of 1-2000 μm, preferably 50-1500 μm, more preferably 100 to 1000 μm in size. The particulate hydrogel may conveniently be prepared by contacting the hydrogel with water and subjecting the swollen hydrogel to shear stress such that it is comminuted to particles, as described in GB-A-2100269.

By incorporating into an envelope as described in GB-A-2144051 formed compresses according to the present invention, it is possible to obtain a sustained release device having a very high content of active ingredient and accordingly to provide sufficient active ingredient for long periods. For example, to take the illustration above, if the active ingredient/hydrogel containing composition of original average density $0.25\text{g.cm}^{-3}$ is replaced by a compressed composition of average density $0.8\text{g.cm}^{-3}$, sufficient active ingredient for 200 days can be fitted in to a hard gelatin capsule of sufficient size for administration to cattle.

The envelope may contain a single formed hydrogel compress or may contain more than one such compress. The shape of the compress(es) will generally depend on the most convenient shape for administration and/or on the convenience of manufacture. For example hemicylindrical compresses are generally convenient for administration, since two fit conveniently in a cylinder for administration. However, hemicylinders may be inconvenient as requiring a special press. Thus it may be preferred to include a plurality of compressed hydrogel tablets in the envelope.

When such an envelope containing compress(es) according to the present invention is introduced into, for example, the stomach of an animal or human patient, there is a rapid initial increase in size of the envelope because of the disintegration of the formed compress(es) and the returning of the previously compressed hydrogel particles to their original size. This prevents immediate discharge of the envelope from the stomach. There is then a further increase in size because of the absorption of further water by the hydrogel to give a final substantially-rigid structure.

The ratio of active ingredient to hydrogel will vary according to the degree of swelling required and the amount of active ingredient needed for the desired treatment regime. For example, for a hydrophobic ingredient there may be used a composition comprising substantially 50% by weight hydrogel and 50% by weight active ingredient.

The compresses used can include for example adjuvants, fillers, excipients and flavourings as conventional in such compositions. In the case of water-insoluble active ingredients, surface-active agents may be included to assist dissolution of the active ingredient, if that is desired. According to a feature of the invention, therefore the biologically-, veterinarily- or pharmaceutically-active ingredient is water-insoluble and the device also contails surface-active agents to assist release of the active ingredient. Sustained release of the surface-active agent may be required, in which case the surface-active agent may itself be included within the hydrogel particles or mixed therewith.

Indeed the inclusion of particles of non-hydrogel, non-biologically active material can increase disintegration rates. According to a feature of the invention the compress or compresses also contain particles of non-hydrogel, non-biologically active material.

The compress or compresses may for example contain 5 to 100% by weight of hydrogel.

The present invention is of broad applicability in the formulation of biologically-, veterinarily- or pharmaceutically-active substances releasable at a sustained rate. Examples of classes of biologically-active substances which may be incorporated in the sustained release devices of the present invention include flavourings, pharmaceuticals, bacteriostats, viriscides, veterinary agents such as anthelmintics, pesticides such as insecticides, nematicides, molluscicides and larvicides, herbicides, fungicides, algicides, topical or dermatological agents, antifoulants for marine growth prevention, proteins, for example enzymes, peptides, microbiological and plant hydroculture salts and nutrients and preservatives, veterinary trace metal formulations, and other growth-promoting factors used in animal husbandry: for example, antianaemia preparations and anabolic steroids. Of particular interest are devices comprising at least one pharmaceutical.

The devices of this invention thus find wide application in medical and surgical, including veterinary, contexts and in horticulture and agriculture as well as outside these areas.

Specific classes of drug which may be utilised in the sustained release devices of the invention include abortifacients such as prostaglandins, hypnotics, sedatives, tranquilisers, antipyretics, anti-inflammatory agents, preparations for the treatment of allergies, for example anti-histamines, anti-tussives, anticonvulsants, muscle relaxants, anti-tumour agents, for example those for the treatment of malignant neoplasia, local anaesthetics, anti-Parkinson agents, topical or dermatological agents, diuretics, for example those containing potassium, such as potassium iodide, preparations for the treatment of mental illness, for example preparations containing lithium for use in the treatment of manic depression or containing prostaglandins for the treatment of schizophrenia, anti-spasmodics, anti-ulcer agents, preparations containing various substances for the treatment of infection by pathogens including anti-fungal agents, for example metronidazole, anti-parasitic agents and other anti-microbials, anti-malarials, cardiovascular agents, preparations containing hormones, for example androgenic estrogenic and progestational hormones, notably steroids such as oestradiol, sympathomimetic agents, hypoglycaemic agents, contraceptives, nutritional agents, preparations containing enzymes of various types of activity, for example chymotrypsin, preparations containing analgesics, for example aspirin, peptides e.g. luteinising hormone, releasing hormone, oxytocin, growth-releasing hormone, antibodies and anti-iodiotypic antibodies, antibiotics such as sulphonamides, penicillins, tetracyclins, cephalosporins, trimethoprin, clavulanic acid, hormones, e.g. melatonin, insulin, growth hormones, placental lactogen, testosterone, oestradiol, medroxyprogesterone, vitamins, e.g. vitamin C, folic acid, prostaglandins, anti-inflammatories e.g. corticosteroids (dexamethasone), aspirin, phenylbutazone, and other non-steroidal anti-inflammatories, minerals e.g. iron, zinc, gastro-intestinal drugs e.g. bismuth and aluminium salts and cimetidine, and agents with many other types of action including nematocides and other agents of veterinary application. Mixtures of active substances may be incorporated into the sustained release compositions.

The sustained release devices of this invention may be used as contraceptive devices suitably containing, as active substance, at least one natural or synthetic steroid sex hormone for example an oestrogen or progestogen. Suitably progestogens include the natural progesterone and its synthetic analogues, including 11-dehydroprogesterone, delalutin, 21-fluoro-17-acetoxy-6-$\alpha$-methylprogesterone, medroxyprogesterone acetate, megestrol acetate, chlormadinone acetate, ethisterone, dimethisterone, A-norprogesterone, 19-norprogesterone, 21-norprogesterone, normethandrone, norethynodrel, norethindrone and its acetate, DL- and D-norgestrel, norgestrienone, ethynodiol diacetate, lynstrenol, ethynylestradiol, retroprogesterone, dydrogesterone, norvinodrel, quingestranol acetate, norethisterone and its acetate and oenanthate, anagesterone acetate, medrogestone, clomagestone, allyl estrenol and cingestol, preferably progesterone. Suitably oestrogens include the natural $\beta$-oestradiol and its synthetic analogues, principally ethinyloestradiol or mestranol, preferably $\beta$-oestradiol.

The sustained release devices of this invention are also useful in the treatment of diabetes and pernicious anaemia where, for example, the controlled release of insulin and cobalmin, respectively, may be utilised.

Moreover, the sustained release devices of this invention are particularly suited to treatment, both prophylactic and therapeutic, of tropical diseases; for example malaria, leprosy, schistosomiasis and clonorchiasis. Examples of drugs which can be used as pharmaceutically-active substance in sustained release devices of this invention for the treatment of these and other tropical diseases include quinine, sulphonamides, rifamcin, clofazimine, thiambutosine, chlorphenyl derivatives, chlorguamide, cycloguanil, pyrimethamine, sulphadiazine, trimethoprim, quinoline derivatives such as pamaquine, chloroquine, pentaquine, dapsone, sodium sulphoxone, sulphetrone, sodium hydnocarpate and sodium chaulmoograte. Drugs of particular effectiveness are cycloguanil, pyrimethamine and sulphadiazine.

Anti-biotics, such as tetracycline (both as free base and hydrochloride or a mixture thereof), have also been found to be efficacious in the treatment of tropical disease in combinations according to this invention.

The sustained release devices of this invention are also very well suited to veterinary applications. Examples include anthelmintic preparations, liquid depot preparations of antibiotics for general antibacterial activity and also in the treatment of anaplasmosis in cattle; preparations for provision of a wide spectrum of activity against both ectoparasites, for example termites and endoparasites including arthropods, arrested larvae stages of nematodes, lungworms and general strongyles: these may comprise avermectins; preparations for provision of activity against tremotode, cestode and roundworm infections: these may comprise amoscanate and praziquantel: preparations for provision of activity against theileria in cattle: these may comprise biologically-active naphthoquinones such as menoctone; preparations for provision of activity against babesiosis in cattle, horses and dogs: these may comprise berenil, amidocarb and diampron; preparations for provision of activity against liver fluke in sheep and cattle and against <u>Haemonchus</u> species: these may comprise closantel.

In accordance with a particularly preferred feature of this invention, there is provided a pro-drug composition wherein at least part of the organic compound (a) comprises a mono-, di- or poly-carboxy, hydroxy or mercapto-substituted biologically-active compound.

Examples of such biologically-active compounds include hydroxylated steroids, such as norethisterone or laevo-norgestrel; prostaglandins, such as $PGE_1$, $PGF_1\alpha$, $PGE_2$, $PGF_2\alpha$, $PGE_3$, $PGF_3$ $\alpha$ , 15-methyl $PGF_2$, 16,16-dimethyl-$PGE_2$, 16-phenoxy-17,18,19,20-tetramer-$PGE_2$, 16,16-dimethyl-<u>trans</u>-$\Delta^2$-PGE, and 16-(3-trifluoro methyl phenoxy)-17,18,19,20-tetranor-$PGF_2\alpha$; and acetylsalicylic acid. Certain of the previously mentioned substituted hydrocarbons are themselves biologically active; for example alkyl phenols.

The walls of the envelope according to the present invention may be of any suitable flexible water-permeable or porous perforated plastics, knitted, braided or woven material with the seams secured as described in GB-A-2144051. It must of course be sufficiently tough to remain intact during use. Any pores or holes must not be large enough to permit unintentional escape of the envelope contents either in the dry unswollen state or in the swollen state.

After all the active ingredient is released the envelope is either retained in the stomach, if this is acceptable, or it can be made of material such that it eventually degrades within the stomach, or the envelope may have stitching or sutures which degrade and release the remaining contents and thus the envelope and its remaining contents are discharged as described in GB-A-2144051.

For administration, the envelope may conveniently be held in a container which will rapidly decompose in the rumen, e.g. in a hard gelatin capsule, which opens in the stomach to release the envelope.

The invention is further illustrated with reference to the accompanying drawings in which:

Figure 1 is a section through a capsule containing an envelope according to the present invention suitable for use in the treatment of cattle for example, and

Figure 2 shows the envelope of Figure 1 in swollen form.

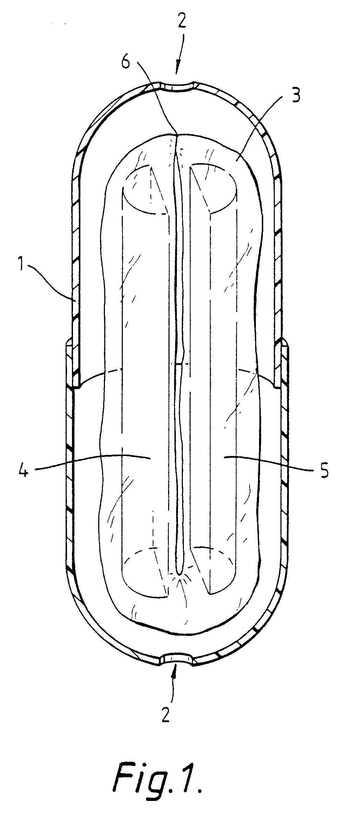

Referring to Figure 1 there is shown a capsule 1 for use in administration of active ingredient to cattle which can for example be 100mm x 38mm. The capsule 1 of water-impermeable material has holes 2 in its wall to allow entrance of water. Within the capsule 1 there is provided an envelope 3 in the form of a tube of flexible water-permeable or porous perforated plastics, knitted, braided or woven material. Within the envelope 3 two hemi-cylindrical shapes 4, 5 formed of compressed hydrogel charged with active ingredient and the ends of the envelope 3 are secured together at 6 to provide the envelope.

Alternative shaped forms can comprise simply compressed hydrogel, hydrogel particles with active ingredient and/or surfactant and/or inert carrier such as silica.

The device of Figure 1 is manufactured by compressing, in a hemi-cylindrical press, a mixture of hydrogel particle and any other ingredient. The mixture is compressed until it retains the formed shape. Two of such hemi-cylindrical formed shapes in the case of Figure 1 are inserted into a tube and the ends of the tube secured together to provide the envelope 3 shown in Figure 1. The envelope is of course made of water-permeable and porous flexible perforated plastics, knitted, braided or woven material as described in GB-A-2144051. The whole is then placed in a capsule, e.g. a conventional capsule as used for administration purposes.

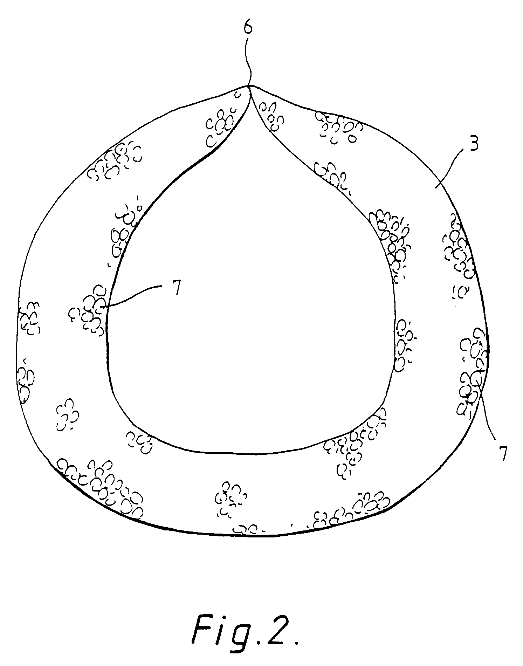

In use the capsule is administered to the patient in conventional way. For example it can be forced down an animal's throat using a bolus gun. When the capsule enters the animal's stomach, water enters the capsule 1 and traverses the walls of envelope 3 causing the formed shapes of hydrogel to disintegrate within the envelope 3 as the hydrogel particles return to their original non-compressed form and the capsule 1 is forced open. Subsequently the hydrogel particles swell further in the stomach with the absorption of water to form the structure shown in Figure 2 with the swollen particles of hydrogel 7 inside.

The manufacture of compositions for use in compresses for the envelopes according to the invention is further illustrated in the following Examples 1 to 7. Example 8 illustrates the compression of hydrogels and Examples 9 onwards illustrate devices according to the present invention.

Methods of preparation of hydrogel

Hydrogel 1

Melted poly(ethylene glycol), Mn 7000-9000, (1,000kg) was thoroughly mixed with crude methylene diisocyanate (79.6g). Distilled water (2.0cm$^3$) was added to the reacting mixture while being mixed for about 40 seconds. The mixed material is then poured into a container and cured for an hour in an oven at 95°C. Solid polymer block is then removed from the container and broken up before placing in a tub of cold water.

After at least an hour the swollen gel is disintegrated, filtered and dried. The dried granules are then sieved through standard sieves to grade different particle size ranges.

Hydrogel 2

Melted poly(ethylene glycol), Mn 8000, is filtered through a porosity 2 sintered funnel and dried under vacuum with a stream of oxygen and moisture-free nitrogen bubbling through it for 5 hours at 95°C.

The melted dried poly(ethylene glycol), from above, (500.0g) was then mixed with the solution of anhydrous ferric chloride in 1,2,6-trihydroxy-hexane (HT) (0.606g $FeCl_3$/15.544g HT) also at 95°C.

3,4-dihydro-2H-pyran-2-methyl-(3,4-dihydro-2H-pyran-2-carboxylate) (90.944g) was then added to the above mixture and stirred for two minutes. The mixture was cured for 4 hours in an oven at 90°C. While the gel is still hot it is cut up into small pieces and allowed to swell in double distilled water for 24 hours.

Thereafter it is disintegrated, filtered and dried. The dried particles are then sieved through standard sieves and graded as granules of different size ranges.

Hydrogel 3

Melted poly(ethylene glycol), Mn 8660, was dried under vacuum with a stream of oxygen and moisture-free nitrogen bubbling through it for five hours at 95°C.

The melted dried poly(ethylene glycol), from above, (1000 g) was mixed with a solution of anhydrous ferric chloride in 1,2,6-trihydroxy hexane (HT)(0.211 g $FeCl_3$/7.737 g HT) also at 95°C.

Bis-cyclohexyl methylene-4,4′-diisocyanate(Desmodur W ex Bayer; 46.983 g) was then thoroughly mixed with the mixture and poured into polypropylene moulds preheated to 95°C, and stored for four hours in an oven at 95°C. The polymer obtained was cooled to ambient temperature and swollen in water. Thereafter it was disintegrated, filtered and dried. The dried particles were then sieved through standard sieves and graded as granules of different size ranges.

EXAMPLE 1

Tablets were compressed using an infra-red press applying a force of 10 tons for two minutes in each compression. A compressed tablet of hydryogel 1 granules of different particle size range was separately placed in a beaker full of water at 37°C. Table 1 indicates the details on disintegration.

7

## TABLE 1

### Disintegration characteristics of Hydrogel 1 Tablets

| Hydrogel 1 Granules | Weight of Tablet | Time for complete disintegration | Observations on Tablet |
|---|---|---|---|
| ($\mu$m) | (g) | (s) | |
| 125–355 | 1.1886 | 36.5 | Floated |
| 355–425 | 0.9169 | 44.8 | Floated |
| 425–699 | 0.8143 | 66.2 | Floated |
| 699–853 | 0.7522 | 15.0 | Sank |
| 699–1003 | 0.7508 | 27.8 | Sank |

EXAMPLE 2

Tablets were prepared and tested as in Example 1 except that there were used Hydrogel 2 granules of different particle sizes. Table 2 displays the details.

## TABLE 2

### Disintegration characteristics of Hydrogel 2 Tablets

| Hydrogel 2 Granules | Weight of Tablet | Time for complete disintegration | Observations on Tablet |
|---|---|---|---|
| ($\mu$m) | (g) | (s) | |
| 250 | 1.1703 | 122 | Floated |
| 355–425 | 1.3162 | 118 | Floated |
| 425–710 | 1.0451 | 104 | Floated |
| 710 | 1.0594 | 69 | Sank |

EXAMPLE 3

Hydrogel 1 granules and calcium carbonate were physically mixed and tablets were prepared and tested as in Example 1. Tables 3-4 provide the details.

## TABLE 3
### Disintegration characteristics of Blend Tablets

| Hydrogel 1/CaCo$_3$ (699-1003$\mu$m) w/w | Weight of Tablet (g) | Time for complete disintegration (s) | Observations on Tablet |
|---|---|---|---|
| 5:95 | 0.8656 | 5 | Sank |
| 25:75 | 1.0076 | 7 | Sank |
| 45:55 | 1.1150 | 9.5 | Sank |
| 65:35 | 1.2444 | 13.2 | Sank |

## TABLE 4
### Distintegration characteristics of Blend Tablets

| Hydrogel 1/CaCo$_3$ (355-425$\mu$m) w/w | Weight of Tablet (g) | Time for complete disintegration (s) | Observations on Tablet |
|---|---|---|---|
| 5:95 | 0.8614 | 36.1 | Sank |
| 25:75 | 1.0507 | 5.0 | Sank |
| 45:55 | 1.2746 | 8.0 | Sank |
| 65:35 | 1.2114 | 5.5 | Sank |

EXAMPLE 4

Hydrogel 1 granules (699-853 μm) were charged from a 25% w/w solution of potassium bromide in water. The granules were left to swell in the solution over two days before filtering and subsequent drying. The dried material is called Hydrogel 1 loaded with potassium bromide. Tablets from this material were prepared and tested as in Example 1. Table 5 indicates the disintegration details.

## TABLE 5

### Disintegration characteristics of a Tablet from
### Hydrogel 1 loaded with KBr

| Hydrogel 1 loaded with KBr | Weight of Tablet (g) | Time for complete disintegration (s) | Observations on Tablet |
|---|---|---|---|
| One Tablet | 1.0609 | 5.5 | Sank |

EXAMPLE 5

Hydrogel 1 granules (699-853 μm) were swollen in dilute aqueous solution of the dye Blue Dye (DUASYN BLUE AE) of Hoechst. The granules were filtered, dried and compacted into tablets as before.
The tablet obtained (0.5804g) disintegrated completely in 19 seconds when placed in water at 37°C.

EXAMPLE 6

Hydrogel 1 granules (699-853 μm) were charged from a 5% solution of Griseofulvin in chloroform. The dried material was compacted into tablets as usual and tested.
A tablet (0.6569g) completely disintegrated into constituent tiny particles in 27 seconds when placed in distilled water at 37°C.

EXAMPLE 7

Tablets were compressed using an infra-red press applying a force of ten tons for two minutes for each compression. Compressed tablets of Hydrogel 3 granules of different particle size ranges were separately placed in beakers full of water at 37°C. Table 6 below indicates the details on disintegration.

## TABLE 6

### Disintegration characteristics of Hydrogel 3 Tablets

| Hydrogel 3 Granules ($\mu$m) | Weight of Tablet (g) | Time for complete disintegration (s) | Observations on Tablet |
|---|---|---|---|
| 700 | 1.0172 | 180 | Sank immediately |
| 700 | 1.0223 | 170 | Sank immediately |
| 710-1003 | 1.0096 | 100 | Floated for 30 seconds and sank |
| 710-1003 | 1.0163 | 90 | Floated for 30 seconds and sank |

EXAMPLE 8

Hydrogel 1 granules were compressed, using a stainless steel mould, to a hemicylinder. This hemicylinder disintegrated rapidly when placed in water. The following are the details of the machine and mould used.

Mould consists of two parts, "Punch" and "Die "Punch" dimensions (100 x 25 x 70)mm with usable depth of 70mm.

"Die dimensions (101 x 25.1 x 12.5)mm with 12.5 mm as the radius at the base and usable depth of 70mm.

Compressing Machine:

Avery Denison 60 ton hydraulic press with digital control.

Force used for compacting hydrogel into hemicylinders - 110 kN

During compression the compression force increases from zero to a final value of 10.1 tons over 2 minutes. The entering speed of "Punch" into "Die" was 25mm/min.

EXAMPLE 9

Fenbendazole (40 g; ex Hoechst) was gradually dissolved in a methyl alcohol/concentrated hydrochloric acid mixture (20 cm$^3$; 90:10 volume/volume). Hydrogel 1 (20 g; particle size 500-600$\mu$m) was added to the fenbendazole solution and the mixture stirred. After approximately 20 minutes, the fenbendazole-loaded hydrogel particles were removed by filtration under vacuum. The loaded polymer was then sieved through a fine mesh to remove unassociated drug particles. The final particles contained an average of 47.2% by weight fenbendazole.

32 g of the loaded fenbendazole hydrogel particles and 8 g unloaded Hydrogel 1 particles (255-500 $\mu$m) were mixed together. This mixture was approximately divided and each half was moulded into a cylinder using the apparatus of Example 8. The hemicylinder moulds were cooled to assist removal of the moulded hemicylinders. Two of these hemicylinders were then incorporated into a device as illustrated in Figures 1 and 2 of the accompanying drawings. Thus the device contained 40 g of loaded and unloaded hydrogel with an approximate content of 16 g fenbendazole.

11

EXAMPLE 10

Fenbendazole as used in Example 9 (40 g) was gradually dissolved in a methyl alcohol/concentrated hydrochloric acid mixture (200 cm³; 90:10 volume/volume) polyoxyethylene (20) sorbitan monolaurate (Tween 20, ex Aldrich Chemicals; 40 g) were added to the solution to form a compatable mixture.

Hydrogel 1 (20g: particle size 500 to 600 μm) were added to the fenbendazole/Tween 20 mixture and were left to swell in the mixture for about one hour before the swollen particles were filtered off.

The particles were then placed in a freezer at -18°C for an hour to freeze the Tween 20. The particles were then washed three times (3 x 100 cm³) with petroleum ether (60-80°C boiling point); the petroleum ether being left in contact with the particles for at least two minutes before being removed by suction.

The loaded particles were then dried in a vacuum oven at room temperature to constant weight.

The final particles contained an average of 59.9% by weight of the fenbendazole/Tween 20 mixture. By weighing after extraction of Tween 20 in water and comparison with the original unloaded granules it was estimated that the loaded particles comprised approximately 31-32% fenbendazole, 25%-26% of Tween 20 and 43% hydrogel.

50 g of the fenbendazole and Tween 20 loaded hydrogel particles were compressed into two hemicylinders as described in Example 9; each hemicylinder containing approximately 25 g. Two of these hemicylinders were then incoporated into a device as described with reference to Figures 1 and 2 of the accompanying drawings. The device thus obtained contained approximately 16g fenbendazole.

EXAMPLE 11

Tween 20 as used in Example 10 (600 cm³) was dissolved in chloroform (400 cm³). Hydrogel 1 (100 g; 600-700 μm) was added to the Tween 20 solution and left to swell in it for one hour before being filtered off. The loaded particles thus obtained were then placed in a freezer (at -18°C) for one hour and then washed with petroleum ether as described in Example 10.

The final particles contained an average of 49.2% by weight Tween 20.

32g fenbendazole-loaded hydrogel obtained as described in Example 9, and 8g of Tween-loaded hydrogel, were mixed and formed into two hemicylinders, each of approximately 20g, as described in Example 9. These were then loaded into a device as described with reference to Figures 1 and 2 to provide a device containing approximately 16g fenbendazole.

EXAMPLE 12

16g fenbendazole-loaded hydrogel as obtained in Example 9, 8g of fenbendazole powder, and 16g of Tween-loaded hydrogel obtained as decribed in Example 11 were thoroughly mixed.

The mixture was then formed into two hemicylinders by the method described in Example 9; each hemicylinder weighing approximately 20g. The two hemicylinders were then incorporated into a device as described with reference to Figures 1 and 2 of the accompanying drawings; the device thus containing approximately 16g fenbendazole.

EXAMPLE 13

16g fenbendazole loaded hydrogel as obtained in Example 9, 8g of fenbendazole powder and 16g unloaded hydrogel 1 (255-500μm) were thoroughly mixed and formed into two hemicylinders (each of approximately 20g) as described in Example 9. The two hemicylinders were then incorporated into a device as described with reference to Figures 1 and 2 of the accompanying drawings containing approximately 16g fenbendazole.

**Claims**

1.  A device for sustained release of active ingredient, which device comprises an envelope having flexible water-permeable or porous walls and containing swellable material and active ingredient, characterised in that the envelope has flexible water-permeable and porous walls of perforated plastics, knitted, braided or woven material and contains one or more water-disintegratable, self-coherent compresses of hydrogel particles and contains a sustainedly-releasable biologically-, veterinarily- or pharmaceutically-active ingredient.

**2.** A device according to claim 1, wherein the compress or compresses contain a biologically-, veterinaily- or pharmaceutically-active ingredient, the active ingredient being in physical mixture with the hydrogel particles or being chemically bonded to or charged into the matrix of the hydrogel particles.

**3.** A device according to claim 1 or 2, wherein the biologically-, veterinarily- or pharmaceutically-active ingredient is water-insoluble and which also contains surface-active agent to assist release of the active ingredient.

**4.** A Device according to any one of the preceding claims, wherein the compress or compresses also contain particles of non-hydrogel, non-biologically-active material.

**5.** A device according to any one of the preceding claims, wherein there are used hydrogel particles which, unswollen and uncompressed, have a particle size of 50 to 1500 µm.

**6.** A device according to any one of the preceding claims, wherein the compress or compresses contain 5 to 100% by weight of hydrogel.

**7.** A device according to any one of the preceding claims, wherein the hydrogel used is a crosslinked polyethylene glycol.

**8.** A device according to any one of the preceding claims, wherein the envelope has a given maximum non-stretched internal volume and the volume of the quantity of hydrogel present, when fully swollen at 20°C, amounts to at least 66% of the given maximum non-stretched internal volume, but insufficient when fully swollen at 20°C to rupture the envelope

**9.** A device according to claim 8, wherein the quantity of hydrogel when fully swollen at 20°C amounts to at least 100% of the given maximum non-stretched internal volume.

**10.** A device according to any one of the preceding claims, wherein the compress or compresses are made by a method which comprises compressing in a mould the hydrogel particles in admixture with the other ingredients being used.

**11.** A device according to claim 10, wherein the ratio of the uncompressed hydrogel to that after compression (compression ratio) used ranges from 3:1 to 5:1.

## Patentansprüche

**1.** Vorrichtung für die verzögerte Freigabe eines aktiven Bestandteils, wobei die Vorrichtung eine Hülle mit biegsamen, wasserdurchlässigen oder porösen Wänden aufweist und quellbares Material und den aktiven Bestandteil enthält, dadurch gekennzeichnet, daß die Hülle biegsame, wasserdurchlässige und poröse Wände aus perforiertem Kunststoff, gewirktem, geflochtenem oder gewobenem Material aufweist und eine oder mehrere, in Wasser zerfallbare, selbst zusammenhängende Preßkörper von Hydrogelteilchen enthält und einen verzögert freisetzbaren biologisch, veterinärmedizinisch oder pharmazeutisch aktiven Bestandteil aufweist.

**2.** Vorrichtung nach Anspruch 1, dadurch gekennzeichnet, daß der oder die Preßkörper einen biologisch veterinärmedizinisch oder pharmazeutisch aktiven Bestandteil enthalten, wobei der aktive Bestandteil in physikalischem Gemisch mit den Hydrogelteilchen vorliegt oder chemisch an die Matrix der Hydrogelteilchen gebunden oder die Matrix damit beladen ist.

**3.** Vorrichtung nach Anspruch 1 oder 2, dadurch gekennzeichnet, daß der biologisch, veterinärmedizinisch oder pharmazeutisch aktive Bestandteil wasserunlöslich ist und sie auch oberflächenaktives Mittel enthält, um die Freisetzung des aktiven Bestandteils zu unterstützen.

**4.** Vorrichtung nach einem der vorhergehenden Ansprüche, dadurch gekennzeichnet, daß der oder die Preßkörper auch Teilchen von nicht-Hydrogel, nicht biologisch aktivem Material enthalten.

**5.** Vorrichtung nach einem der vorhergehenden Ansprüche, dadurch gekennzeichnet, daß Hydrogelteilchen verwendet werden, die in ungequollenem und nichtverpreßtem Zustand eine Teilchengröße von 50 bis

EP 0 339 035 B1

1500 µm haben.

**6.** Vorrichtung nach einem der vorhergehenden Ansprüche, dadurch gekennzeichnet, daß der oder die Preßkörper 5 bis 100 Gew-% Hydrogel enthalten.

**7.** Vorrichtung nach einem der vorhergehenden Ansprüche, dadurch gekennzeichnet, daß das verwendete Hydrogel ein vernetztes Polyethylenglykol ist.

**8.** Vorrichtung nach einem der vorhergehenden Ansprüche, dadurch gekennzeichnet, daß die Hülle ein gegebenes Maximum an nichtgestrecktem Innenvolumen hat und das Volumen der Menge an vorhandenem Hydrogel, in vollständig gequollenem Zustand bei 20°C, wenigstens 66% des gegebenen maximalen nichtgestreckten Innenvolumens beträgt, jedoch nach voller Quellung bei 20°C unzureichend ist, um die Hülle zu zerreißen.

**9.** Vorrichtung nach Anspruch 8, dadurch gekennzeichnet, daß die Menge an Hydrogel in voll gequollenem Zustand bei 20°C wenigstens 100% des gegebenen maximalen ungestreckten Innenvolumens beträgt.

**10.** Vorrichtung nach irgendeinem der vorhergehenden Ansprüche, dadurch gekennzeichnet, daß der oder die Preßkörper nach einem Verfahren hergestellt sind, das das komprimieren der Hydrogelteilchen in Mischung mit den anderen verwendeten Bestandteilen in einer Form umfaßt.

**11.** Vorrichtung nach Anspruch 10, dadurch gekennzeichnet, daß das Verhältnis des verwendeten nichtverpreßten Hydrogels zu dem nach Verpressung (Kompressionsverhältnis) von 3:1 bis 5:1 reicht.

## Revendications

1.- Dispositif pour une libération prolongée d'un ingrédient actif, lequel dispositif comprend une enveloppe ayant des parois flexibles perméables à l'eau ou poreuses et contenant une substance gonflable et un ingrédient actif, caractérisé en ce que l'enveloppe a des parois flexibles perméables à l'eau et poreuses d'une matière plastique perforée, tricotée, tressée ou tissée et qu'elle contient un ou plusieurs comprimés autocohérents et désintégrables dans l'eau de particules d'hydrogel et contient un ingrédient actif du point de vue biologique, vétérinaire ou pharmaceutique, pouvant être libéré de manière prolongée.

2.- Dispositif selon la revendication 1, dans lequel le(s) comprimé(s) contient(contiennent) un ingrédient actif d'un point de vue biologique, vétérinaire ou pharmaceutique, l'ingrédient actif étant mélangé physiquement aux particules d'hydrogel ou étant chimiquement lié à la matrice de particules d'hydrogel ou chargé dans cette matrice.

3.- Dispositif selon la revendication 1 ou la revendication 2, dans lequel l'ingrédient actif d'un point de vue biologique, vétérinaire ou pharmaceutique est insoluble dans l'eau, le dispositif contenant également un agent tensio-actif pour faciliter la libération de l'ingrédient actif.

4.- Dispositif selon l'une quelconque des revendications précédentes, dans lequel le(s) comprimé(s) contient(contiennent) également des particules d'un produit non biologiquement actif et non sous la forme d'hydrogel.

5.- Dispositif selon l'une quelconque des revendications précédentes, dans lequel on utilise des particules d'hydrogel qui, non comprimées et non gonflées, ont une dimension de particules de 50 à 1500 µm.

6.- Dispositif selon l'une quelconque des revendications précédentes, dans lequel le(s) comprimé(s) contient(contiennent) 5 à 100% en poids d'hydrogel.

7.- Dispositif selon l'une quelconque des revendications précédentes, dans lequel l'hydrogel utilisé est un polyéthylène glycol réticulé.

8.- Dispositif selon l'une quelconque des revendications précédentes, dans lequel l'enveloppe a un volume intérieur maximal donné à l'état non étiré et le volume de la quantité d'hydrogel présent, lorsqu'il est totalement gonflé à 20°C, est égal à au moins 66% du volume intérieur maximal donné à l'état non étiré, mais qu'il est insuffisant, lorsqu'il est totalement gonflé à 20°C, pour rompre l'enveloppe.

9.- Dispositif selon la revendication 8, dans lequel la quantité d'hydrogel, lorsqu'il est complètement gonflé à 20°C, est égale à au moins 100% du volume intérieur maximal donné à l'état non étiré.

10.- Dispositif selon l'une quelconque des revendications précédentes, dans lequel le(s) comprimés est-(sont) préparé(s) par un procédé qui consiste à comprimer dans un moule les particules d'hydrogel en mélange avec les autres ingrédients utilisés.

11.- Dispositif selon la revendication 10, dans lequel le rapport de l'hydrogel non comprimé à l'hydrogel après compression (taux de compression) est compris entre 3:1 et 5:1.

14

Fig.1.

Fig.2.